# EUROPEAN PATENT APPLICATION

(11) **EP 4 542 557 A1**
(43) Date of publication of application: **23.04.2025**
(21) Application number: 23203695.4
(22) Date of filing: 16.10.2023
(51) Int. Cl.: G16H 20/10, G16H 40/67, G16H 50/50

(54) **METHOD FOR DETERMINING EFFICACY OF A MEDICAL TREATMENT**

(71) Applicant: Koninklijke Philips N.V., 5656 AG Eindhoven (NL)
(72) Inventor: GERHARDT, Lutz Christian, Eindhoven (NL); JOHNSON, Mark Thomas, Eindhoven (NL); VAN LIESHOUT, Ron Martinus Laurentius, Eindhoven (NL)
(74) Representative: Philips Intellectual Property & Standards

(57) **Abstract**

There is described a computer implemented method (100) for determining efficacy of a medical treatment based on usage of a personal care appliance (402), such as an oral care device; e.g., toothbrush. The method comprises receiving (101) user-related data comprising a time of administration of the medical treatment, receiving (102) sensor data (t1-14) from the personal care appliance captured during usage of the personal care appliance, and a usage time of the personal care appliance, mapping (103) the received sensor data to an efficacy model corresponding to the medical treatment, based on the received user data and usage time of the personal care appliance, determining (104) efficacy of the medical treatment based on the mapping.

## Description

### FIELD OF THE INVENTION

The subject-matter of the present disclosure relates to a method of determining efficacy of (and/or adherence to) a medical treatment; in particular, a method of determining efficacy of (and/or adherence to) a medical treatment based on usage of a personal care appliance. Yet more specifically, the method may be a computer implemented method.

### BACKGROUND OF THE INVENTION

Incorrect administration of a medical treatment - e.g., due to incorrect regularity, incorrect dosage, etc - has a negative impact not only on (longer-term) treatment success of an underlying primary disease, but can also negatively affect other activities such as personal care routines, particularly daily oral care; especially if medication is required to control physical activity and mobility, cognitive and visual coordination associated with the person's underlying disease.

If treatment application and efficiency is not controlled, this may also promote worsening of the primary disease or foster development of co-morbidities or oral-systemic disease complications. Furthermore, monitoring treatment administration and compliance to medication is difficult in an unsupervised home setting and relies purely on a unregular personal patient-GP communication.

It is therefore now highly desirable to monitor medical treatment adherence and efficiency in a home setting.

### SUMMARY OF THE INVENTION

The present invention is defined according to the independent claims. Additional features will be appreciated from the dependent claims and the description herein. Any embodiments which are described but which do not fall within the scope of the claims are to be interpreted merely as examples useful for a better understanding of the invention.

In particular, embodiments of the invention relate to a technique to monitor medical treatment adherence and efficiency during person hygiene care routines, such as daily oral routine, in a convenient, automatic and unobtrusive manner: to obtain insights whether treatment (e.g., drug medication) still works and/or whether there might be reason to change an aspect of the treatment such as dosage, drug type, in intake regimen, or oral care regimen.

Suitably, in one aspect of the present invention there is provided a computer implemented method for determining efficacy of a medical treatment based on usage of a personal care appliance, such as an oral care device (e.g., powered toothbrush). The method comprises receiving user-related data comprising a time of administration of the medical treatment, receiving sensor data from the personal care appliance captured during usage of the personal care appliance, and a usage time of the personal care appliance, mapping the received sensor data to an efficacy model corresponding to the medical treatment, based on the received user data and usage time of the personal care appliance, determining efficacy of the medical treatment based on the mapping.

Advantages of the present invention include: unobtrusive monitoring of medication intake and compliance; re-assurance that medicine is being taken (both for the user and potentially advising medical practitioner); cost-saving & resource-efficient assurance that medication is taken and effective along prescription (less or no need to rely on multiple, regular double-checks by medical staff, e.g. blood sampling, measurement with additional home devices such as wrist-worn watch with inertial measurement unit; IMU); increase convenience and engagement with personal care (e.g., support compliance to oral care by providing feedback on drug effectiveness).

In an example, determining treatment efficacy comprises verifying administration of the medical treatment occurred. In another example, determining treatment efficacy comprises determining treatment effect. In an example, the result of determining the efficacy may be transmitted to a server, so as to be accessible by both the user and medical professionals, or even insurances. In an example, the method may comprise controlling a display to output a result of determination of the efficacy. In an example, the efficacy model may be requested, and subsequently received, from a server, ready for mapping to the received sensor data. In an example, the time of administration may be determined by a dispensing time given by an automatic medication (treatment) dispenser.

In one example, the sensor data comprises IMU data captured by an inertial measurement unit, and mapping the received sensor data to the efficacy model comprises utilising the IMU data as a proxy parameter for a parameter in the efficacy model. Suitably, conditions such as joint/finger stiffness, may be analysed from such IMU data and provide indication on different hand grip patterns or changing user behaviour, e.g. used as proxy for detecting finger joint stiffness and the effect of medical treatment on relieving joint stiffness.

In one example, the sensor data comprises data captured by one or more of a fluid sensors, a galvanic skin response sensor, an image sensor or multi-hyperspectral camera, a temperature sensor, and an electrogram, any (chemical) sensor to detect concentrations of an administered drug/medication, or any parameter of the efficacy model. Here mapping to the efficacy model may be direct mapping using the received data (i.e., the data is directly used in the relevant efficacy model).

In an example, the medical treatment comprises a pharmacological treatment, and the efficacy model comprises a predetermined pharmacometrics model, such as a pharmacokinetics curve, a pharmacodynamics curve, or a combination thereof. Here, the mapping comprises determining at least one of a ratio of measured to expected pharmacological concentration or a ratio of measured to expected pharmacological effect, and determining the efficacy of the medical treatment comprises determining whether the ratio satisfies a predetermined threshold.

In an example, the user-related data comprises a first time corresponding to a first administration of the medical treatment for a predetermined time period, and a second time corresponding to a subsequent administration of the medical treatment for a predetermined time period. Here the mapping may comprise determining a change in therapeutic effect between the first time and the second time. In this way treatment response effectiveness may be readily measured and used as a metric in determination of e.g., treatment changes, etc.

In one example, receiving the sensor data from the personal care device may comprise transmitting a control signal to the personal care device to activate a secondary or auxiliary treatment function of the personal care device, receiving first sensor data corresponding to when the secondary or auxiliary treatment function is not active during usage of the personal care device, and receiving second sensor data corresponding to when the secondary or auxiliary treatment function is active during usage of the personal care device. The mapping may suitably comprise mapping the received sensor data to the efficacy model comprises mapping the first sensor data and second sensor data based on the received user-related data and usage time of the personal care appliance. Determining the efficacy may suitably comprise comparing the mapping of the first sensor data to the mapping of the second sensor data.

Here, device switch-on (e.g. start of regular oral hygiene or automatic setting or mode change detected by a sensor) provides a well-defined time trigger signal for correlation of medication effect curves to enable reliable and robust drug response measurement. Device setting change (e.g. adding an adaptive co-treatment) can furthermore enhance specificity (potentially also sensitivity) of the drug response measurement (e.g. if comorbidities are present and multiple medication is taken such as person with arthritis and tremor) and augment the therapeutic effect and device usability. Also, this example and other methods described in this invention allow for cost-saving & resource-efficient assurance that medication is taken and effective along prescription (less or no need to rely on multiple, regular double-checks by medical staff, e.g. blood sampling, measurement with additional home devices such as wrist-worn watch with IMU).

In another aspect of the present invention, there is provided an electronic apparatus comprising at least one processor configured to carry out the aforementioned method.

In another aspect of the present invention, there is provided a transitory or non-transitory computer readable medium, having instructions stored thereon that, when executed by a processor, cause the processor to perform the aforementioned method.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying figures, in which:
Fig. 1 shows an example computer implemented method for determining efficacy of a medical treatment based on usage of a personal care appliance;
Fig. 2 shows an example of using a pharmacokinetics (PK) based model; Fig. 2A shows an example PK curve; Fig. 2B shows a graph of measured to expected concentration; Fig. 2C shows a ratio of measured to expected pharmacological concentration;
Fig. 3 shows an example of using a combined pharmacokinetics-pharmacodynamics (PK/PD) based model; Fig. 3A shows an example PK/PD curve; Fig. 3B shows a graph of measured medication effect; Fig. 3C shows a ratio of measured to expected pharmacological effect;
Fig. 4 shows an example system for performing the methods herein;
Fig. 5 shoes a more detailed method of performing the techniques herein;
Fig. 6 shows another example method building on the method of Fig. 1;
Fig. 7 shows an example mapping use in the method of Fig. 6.

### DESCRIPTION OF EMBODIMENTS

Personal care activities, such as oral care, require sufficient motor control, visual and cognitive coordination to ensure proper cleaning and health outcomes. However, medication or lack of medication can affect these. For example, many (chronic) diseases impact and affect a person's ability to perform proper oral care routine - e.g. along recommended instructions for use or recommendations of dentists - and require regular use of medication to alleviate disease symptoms.

For example, joint disorders (inflammatory/osteo arthritis) or motor disabilities (e.g., tremors, joint stiffness/rigor) can adversely affect coordination activities and thus quality of oral hygiene. Such persons are prescribed medications to alleviate joint pain/stiffness. As another example, cognitive disorders or depression can lead to the result a person does not or only brush randomly for a short time < 2 mins.

Table 1 summarizes a list of relevant (chronic) diseases affecting physical motor, cognitive and visual coordination, the typical treatment for each disease, and their potential (oral) side effects.

**Table 1:**

| Use case: Chronic disease | Worldwide prevalence [%] | Treatment (examples) | Primary therapeutic effect | (Oral) side effects caused by medication |
|---|---|---|---|---|
| Hand/Finger arthritis incl joint stiffness | ≈40-50 % | Anti-inflammatory agent, painkiller | Relieves inflammation and pain | Dry mouth, gum swelling |
| Bruxism and Temporo-mandibular joint disorder (TMJD) | ≈25 % | Anti-inflammatory agent, painkiller | Relieves inflammation and pain | Dry mouth, gum swelling |
| Non-essential and Parkinson tremors | ≈ 1- 5% | β-blocker, Anti-seizure medicines, Tranquilizers | Reduce hand shaking and joint stiffness | Dry mouth, blurred, vision, Fatigue, anxiety |
| Depression | ≈ 5 % | Antidepressants | Relieve from stress, anxiety | Dry mouth, acidic tooth decay |
| Diabetes mellitus | ≈ 9 % (1 of 11) | Insulin | Sugar production lowering/inhibitio n agents | Blurred vision |
| COPD/Asthma | 6-12 % | Bronchodilators, oxygen therapy | Makes breathing easier by relaxing/widening airways | Dry mouth, Hand trembling |
| Cognitive disorders incl. mild dementia | 19 % | Mental function enhancers (Nootropics) | Improve state of mind | Dry mouth, daytime sleepiness, blurry vision |
| Hypertension | 28-34 % | ACE inhibitors, diuretics Angiotensin-2 receptor blockers | Lower bood pressure | Dry mouth, gum swelling and overgrowth |

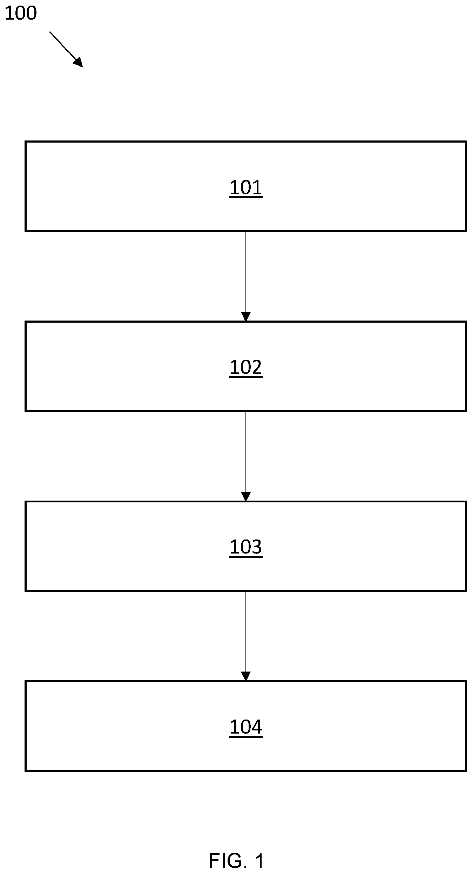

Suitably, by way of introduction, Fig. 1 shows an example computer implemented method 100 for determining efficacy of a medical treatment based on usage of a personal care appliance.

At step 101, the method comprises receiving user-related data comprising time of administration of a medical treatment. The user-related data may more generally be considered as medical data. Suitably, the user related data may comprise information of what the medical treatment is (e.g., a type of drug), administration time of the medical treatment (including actual time of administration and e.g., advised times and/or frequency of application), and also typical schedule for usage of the personal care appliance (e.g., approximate start time, duration, etc), and so on. An example drug usage intake schedule might indicate that medication is taken twice a day (morning and evening), or three times a day (morning, midday, evening). An example personal care appliance schedule might indicate that the personal care routine is performed in the morning and evening (e.g., oral hygiene) or just one or the other (e.g., shaving); it should go without saying that the received data should be relevant to the type of personal care appliance and the usage thereof to which the method is being based.

Suitably, the user-related data may comprise a first time corresponding to a first administration of the medical treatment for a predetermined time period (e.g., over a day), and a second time corresponding to a subsequent administration of the medical treatment for the predetermined time period (e.g., when a second administration during a day occurs).

In this way, the start time of the personal care routine can be used (later in the method) as a well-defined reference time point to time-correlate / sync medication efficacy: for example, medical treatment intake curves and/or medical effect curves, as discussed in more detail below.

At step 102, the method comprises receiving sensor data from the personal care appliance. The sensor data is captured during usage of the personal care appliance so that the captured data may be indicative of a primary therapeutic effect of a medication or a side effect of the medical treatment: for example, the sensor data may be analysed to determine a degree of severity of one or more of the side effects listed in Table 1. Suitably, a usage time of the personal care appliance is also received; preferably the usage time includes at least a start time, but may also include a duration and end time.

In one example, the sensor data may comprise IMU data captured by an inertial measurement unit (which may be provided on the personal care appliance). In another example, the sensor data may comprise data captured by one or more of a fluid sensor, a galvanic skin response sensor, an image sensor or multi-hyperspectral camera, a temperature sensor, and an electrogram, any (chemical) sensor to detect concentrations of an administered drug/medication. The sensor data may comprise IMU data and any other sensor data just listed. It will of course be appreciated that there may be other examples of sensors and their corresponding data, not listed here, which are relevant to the determining of treatment efficacy that could be utilised within the present method.

At step 103, the method comprises mapping the sensor data received at step 102 to an efficacy model corresponding to the medical treatment. The mapping is based on the user related data received at step 101 and the usage time of the personal care appliance received at step 102.

Here the efficacy model may be any suitable model which indicates a person's response to a medical treatment over time. In preferred examples, whereby the medical treatment is pharmacological (i.e., drug based), the efficacy model may be a pharmacometrics model, such as a pharmacokinetics (PK) curve, a pharmacodynamics curve (PD), or a combination thereof (PK/PD). Pharmacokinetic models describe how the body reacts to a drug in terms of absorption, distribution, metabolism, and excretion (ADME). Pharmacodynamic models describe how a drug affects the body by linking the drug concentration to an efficacy, therapeutic effect (or safety) metric. It will be appreciated that the pharmacometrics model will be predetermined based on the medical treatment the user is applying. It will also be appreciated that other clinically validated efficacy models may also be utilised within the present technique.

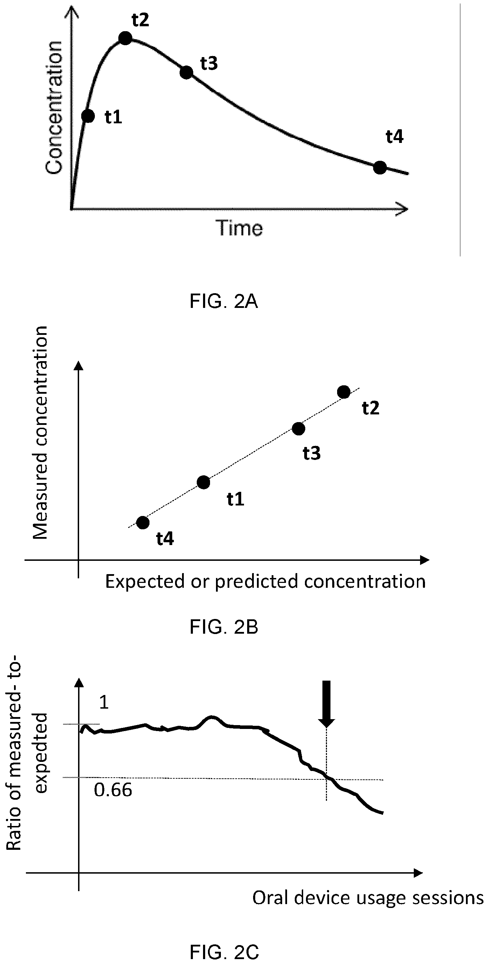

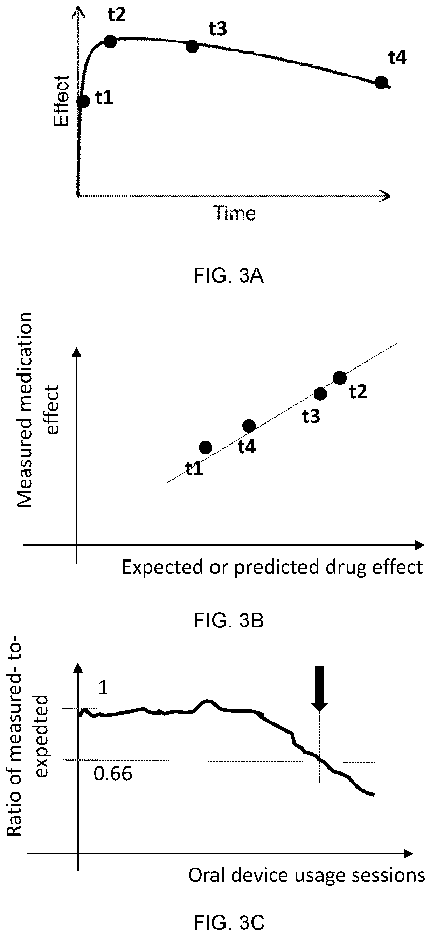

Suitably, in the case of PK, PD, and PK/PD curves, the mapping may comprise mapping the personal care appliance start time and/or duration and/or stop time onto the PK, PD, PK/PD curves. Fig. 2A shows an example of such a mapping to a PK curve at various times (t1-t4) during usage of an appliance. Fig. 3A shows a similar example for mapping to a combined PK/PD curve at various times (t1-t4) during usage of an appliance.

From the curves can be derived graphs for expected (or predicted/forecasted/predetermined) vs actual medication effect, as shown by Figs. 2B and 3B (following from the mapping in Figs. 2A and 3A respectively). The mapping may also comprise determining at least one of a ratio of measured to expected pharmacological concentration, as in Fig. 2C, or a ratio of measured to expected pharmacological effect, as in Fig. 3C (following from Figs. 2B and 2C respectively).

In one example, the mapping may comprise determining a change in therapeutic effect between a first treatment administration time and a second (subsequent) treatment administration time (as received with the user data at step 101). It will be appreciated that, in the case of drugs, treatment concentration lowers during the day and will reach its lowest concentration just before the intake of the next dosage. Because brushing or any oral hygiene usually follows a sequential pattern throughout the day (morning-midday-evening or morning-evening) follows the same pattern it is useful to analyse consecutive medication effectiveness surrogate or direct measurements of the user with respect the therapeutic or sub-therapeutic window.

By way of example, assuming the user is taking medication in the morning (either just before or after brushing teeth), the concentration of the medication will be low and close/on the threshold of the sub-therapeutic window. At that stage the effects, one will see are related to the therapeutics effects (i.e. more trembling, less grip, etc). However, in the evening the concentration will be in the therapeutic window (i.e. the effect related to the therapeutic effect will go down) or above the toxicity threshold (i.e. higher risk of side/adverse effects).

A time-progression curve of consecutive differential values ΔE-M,t (evening minus morning effect at day 1,2, etc) may be used as an indicator or metric for drug response effectiveness. The sign of the relative change (normalised to values between -1, 0 and +1) in combination with the maximum value provides information in which direction the change occurs (e.g. from therapeutic to sub-therapeutic or from therapeutic to toxic).

At step 104, the method comprises determining efficacy of the medical treatment based on the mapping. Suitably determining treatment efficacy may comprise verifying administration of the medical treatment occurred; that is, that the medical treatment was administered at the expected time based on the user related data received at step 101. In the case of a pharmacometrics model, determining the efficacy of the medical treatment comprises determining whether the ratio of measured to expected pharmacological concentration or effect satisfies a predetermined threshold. In an example, the threshold may be if the ratio drops below 0.66 (or 66%) of its initial value.

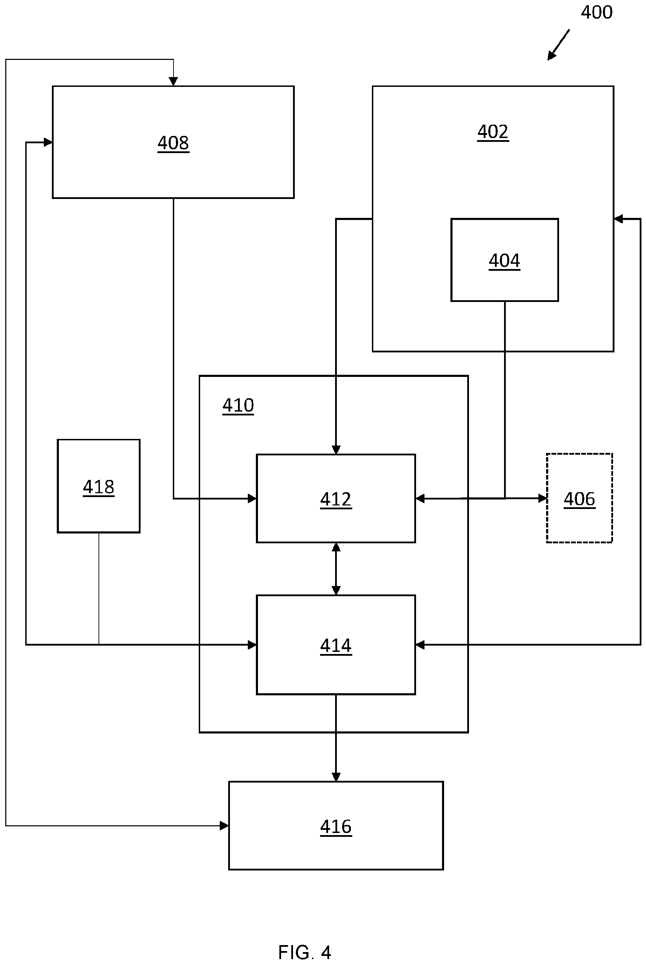

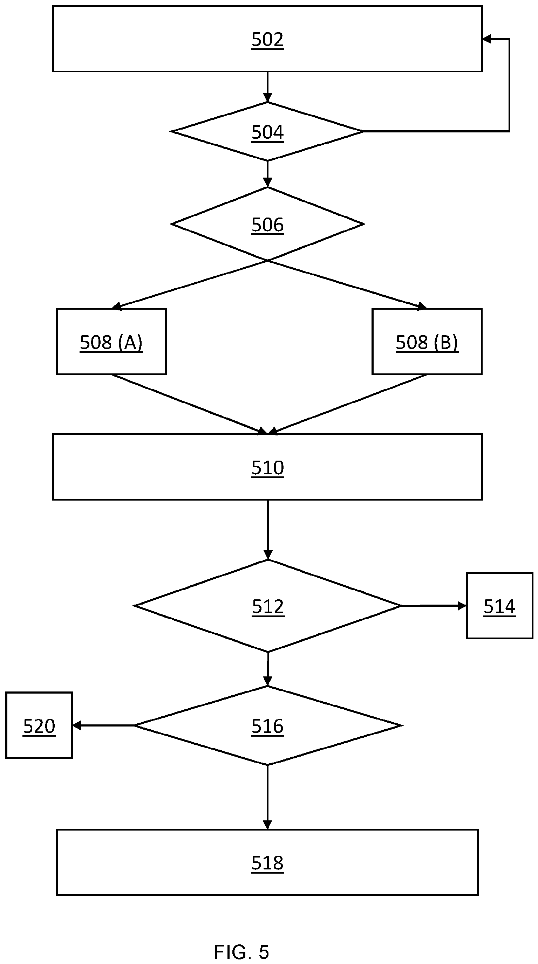

Fig. 4 shows an example system 400 configured to apply the techniques herein in the case of an oral hygiene device 402, while Fig. 5 shows the method of Fig. 1 in more detail as applied to the system 400.

The system comprises the oral device 402, which has on-board sensors 404, optional external sensors 406 (that is, external to the oral device 402), a server 408, an electronic apparatus 410 comprising a processor 412 for example for executing an algorithm and a data communication module 414, a user interface (UI) 416 (integrated in 402 or remote), which may be coupled to the electronic apparatus 410 and/or server 408, and optionally an automatic drug dispenser 418.

Referring now to Fig. 5, at step 502, the method comprises initiating a medication mode in the oral device 402 or in/via a remote accessory such as smart phone App. Initiation may be via input direct to the oral device 402 (e.g., by a button), or may be achieved by suitable input to the apparatus 410 which communicates an activation signal to the oral device 402 via the communication module 414. Initiating the medical mode may cause the activation of a suitable application, or medication program, on the apparatus 410. For instance, specific hardware and software user interfaces can be used to initiate and verify initiation of a medication intake and compliance mode. Once the initiation is successfully verified, the user obtains feedback on the device or Sonicare App. By way of example, hardware may include a toothbrush handle with medication mode or display showing medication icon, while software may include an App with activated medication compliance measurement mode (e.g. add a new tab or activate a greyed-out window).

At step 504, the method comprises validating initiation of the medical mode by the processor 412. If yes (i.e. successfully initiated) the method proceeds to step 506; if no (initiation failed) the system may return to step 502. In an example, step 504 may be considered a check that a suitable app on the electronic apparatus is running and functioning correctly, and if no, the processor waits for the app to function before continuing.

At step 506, the processor receives the user related data comprising time of administration of the medical treatment, as at step 101 (and, of course, the type of medical treatment). The user-related data is received from server 408 (via the communication module 414), being stored, for example, in a suitable database accessible by the user via appropriate authentication, etc. The time of administration received from the database may be updated manually by the user, e.g., via an app on the electronic apparatus 410, or may be obtained automatically, for example from a time of access of automatic drug dispenser 418.

Moreover, the database may comprise, for example, a list of types and number of medications taken by the user, frequency of administration, time schedule, PD-PK model or PD concentration curves of medication, individual specific properties of patient such as age, height, weight, severity of diseases, eating habits, EMR (Electronic Medical Record) data of patient, and so on.

Based on the received user-related data, step 506 also comprises determining (by the processor 412), whether the primary underlying disease affect physicals, cognitive or visual coordination, and then determines a type of sensor data (direct data, proxy parameters, or both) used to determine medication efficacy 514 or medication intake (administration) 512. At step 508, the relevant sensor data is received by the processor 412 via the communication module 414.

In a preferred example, characteristic signatures of proxy parameters from sensors 404 common to many oral devices may be utilised (step 508A). For example, in order to analyse pain or inflammation relief medication to tackle painful mouth opening or impaired grip due to arthritis/TMJ, it is possible to analyse IMU/drive train signatures to determine finger/hand grips related to joint stiffness, mouth opening or brushing patterns to provide feedback on drug intake and effectiveness. Anti-tremor medication intake and effectiveness may be assessed by measuring hand tremor signature picked up by IMU or microphone in oral device. Regular intake/effect of anti-depressants may be assessed by determining that a full brushing session was completed or whether brushing is being systematically omitted.

In other examples direct measurement of drug response (therapeutic effect) may be determined (step 508B) by measurements using any (additional) sensor 404 or measurement in oral device or even external sensor 406 (e.g. part of a healthwatch, or smart phone). For example, the direct measurements could include measurement of concentration of (saliva) biomarkers incl. pH, bad breath gas sensing concentration, galvanic skin response, electrodermal activity, vital signs (blood oxygen, respiration rate, body core temp, heart rate, blood pressure), or image-based measurements e.g. from hyperspectral images incl color using camera's or image sensors.

Table 2 shows sensor data indicative for symptoms related to (chronic) diseases, not limited to but including physical, cognitive and visual coordination.

**Table 2:**

| Use case: Chronic disease | Oral (device) proxy signature or direct measurement parameter indicative for drug effectiveness | Medication /drug |
|---|---|---|
| Hand/Finger arthritis incl joint stiffness | Grip pattern, Brushing pattern | Anti-inflammatory agent, painkiller such as ibuprofen |
| Bruxism and Temporo-mandibular j oint disorder (TMJD) | Mouth opening angle determined from IMU, GSR | Anti-inflammatory agent, painkiller such as ibuprofen |
| Non-essential and Parkinson tremors | Low frequency vibration signature on Sonic frequency, grip pattern | β-blocker, Anti-seizure medicines, Tranquilizers |
| Depression | Brushing duration pattern (length, regularity/consistency) | Antidepressants |
| Diabetes mellitus | Glucose level in saliva alone or in combination with brushing pattern | Insulin |
| COPD/Asthma | Brushing duration pattern (length, regularity/consistency), saturation | Bronchodilators, oxygen therapy |
| Cognitive disorders incl. mild dementia | Brushing duration pattern (length, regularity/consistency) - compliance | Mental function enhancers (Nootropics) |
| Hypertension | Cholesterol biomarker in saliva, PPG signatures to estimate blood pressure | ACE inhibitors, diuretics Angiotensin-2 receptor blocker |

At step 510, the oral device usage time window is mapped onto a predetermined pharmacometrics curve accessible from the database 408 and metrics for drug intake compliance and drug effectiveness are determined.

At step 512, the processor 412 determines the user compliance of the (medication) treatment, i.e. whether the medication has been administered and taken. If it is determined that a treatment regimen is not being adhered to (e.g., a drug hasn't been taken on time), then at step 514 a reminder may be sent to the user via user interface 416. Data indicative of the non-compliance may also be uploaded to the server 408 to update the medical database. In other words, the result of the determining the efficacy of the medical treatment may be transmitted to the server 408. If adherence to the treatment regimen is determined to be "yes", then the method proceeds to step 516.

At step 516, determining the efficacy of the treatment further includes determining whether the treatment is effective based on the calculated metric (over time); e.g., by analysis of the graphs of Figs 2 & 3. If the medication is effective, at step 518 feedback may be provided to the user via the UI 416, and also transmitted to the server 408. If the medication is not effective, at step 520 the method may recommend a change in treatment to the user, via the UI 416 (and may also send data on the recommended change to the server 408).

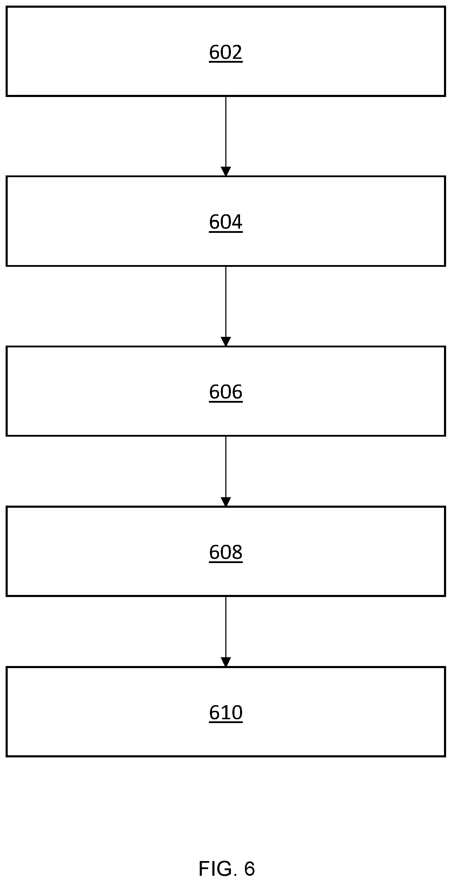

Fig. 6 shows a modification of the above techniques in which an adaptive personal care device with oral device with either tremor reduction or join stiffness treatment function may be used to (selectively) probe the therapeutic medication effect by comparing medication effect measurement with and without addition co-treatment provided by the device.

In this example, known calibration curves or the relationship between device usage time and medication effect (PK-PD curves, or proxy parameter) may be compared for a first state in which an auxiliary treatment function is "on" (e.g., with tremor/heat treatment function active) and a second state in which the auxiliary treatment function is "off" (i.e., without auxiliary treatment).

Suitably, at step 602, step 102 of Fig. 1 may be modified to transmit a control signal to the personal care device to activate the auxiliary treatment function.

At step 604, the method comprises receiving first sensor data from the care device when the auxiliary treatment function is not active. That is, the first sensor data may be considered to correspond to normal usage of the personal care device.

At step 606, the method comprises receiving second sensor data when the secondary or auxiliary treatment function is active (and the personal care appliance is in use).

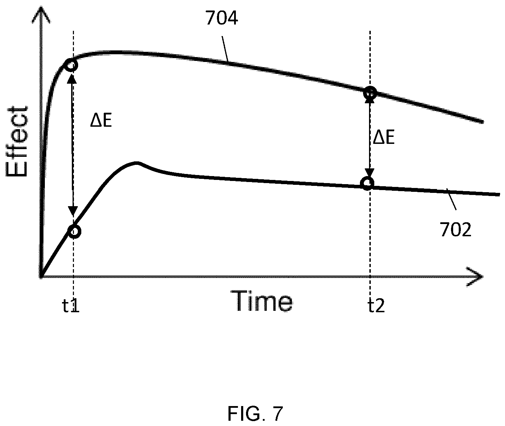

At step 608, the method comprises modified step 103 of Fig. 1 to map both of the first sensor data and second sensor data based on the received user-related data and usage time of the personal care appliance. An example of the mapping is shown by Fig. 7: curve 702 shows the adaptive device "off"; curve 704 shows the adaptive device "on". Suitably, step 610 comprises modifying step 104 to determine the efficacy of the medical treatment based on comparing the mapping of the first sensor data to the mapping of the second sensor data.

By applying a second co-treatment in addition to the use of the medication, we can selectively probe the medication effect by providing additional heat stimulus through a handle with NIR LEDs or tremor reduction by drive train modulation (anti-phase or asymmetric pulse width modulation) and measure the medication effect response before and after adding heat stimulus or activating the tremor reduction mode (Fig. 7).

In practice the personal care device may be programmed to measure in "on" vs "off" duty cycles e.g. 10s on 10 s, and determine the actual effect difference and compared the measured effect difference with the one from databases (known calibration curves). Similar to Figs. 2C & 3C, the ratio of expected vs actual effect size ΔE_{on-off} is used as metric to determine drug response/effectiveness.

In summary, example embodiments of a method for determining efficacy of a medical treatment based on usage of a personal care appliance have been described.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A computer implemented method (100) for determining efficacy of a medical treatment based on usage of a personal care appliance (402), comprising:
receiving (101) user-related data comprising a time of administration of the medical treatment;
receiving (102) sensor data (t1-t4) from the personal care appliance captured during usage of the personal care appliance, and a usage time of the personal care appliance;
mapping (103) the received sensor data to an efficacy model corresponding to the medical treatment, based on the received user data and usage time of the personal care appliance; and
determining (104) efficacy of the medical treatment based on the mapping.

2. The method of claim 1, wherein determining treatment efficacy comprises verifying administration of the medical treatment occurred.

3. The method of any preceding claim, wherein the sensor data comprises IMU data captured by an inertial measurement unit (404), and mapping the received sensor data to the efficacy model comprises utilising the IMU data as a proxy parameter for a parameter in the efficacy model.

4. The method of any preceding claim, wherein the sensor data comprises data captured by one or more of a fluid sensor a galvanic skin response sensor, an image sensor or multi-hyperspectral camera, a temperature sensor, an electrogram, a sensor configured to detect concentrations of an administered drug/medication, or a sensor (406) configured to detect any parameter of the efficacy model.

5. The method of any preceding claim, wherein the medical treatment comprises a pharmacological treatment, and the efficacy model comprises a predetermined pharmacometrics model.

6. The method of claim 5, wherein the predetermined pharmacometrics model comprises at least one or a combination of a pharmacokinetics curve and a pharmacodynamics curve.

7. The method of claim 5 or 6, wherein the mapping comprises determining at least one of a ratio of measured to expected pharmacological concentration or a ratio of measured to expected pharmacological effect, and determining the efficacy of the medical treatment comprises determining whether the ratio satisfies a predetermined threshold.

8. The method of any preceding claim, wherein the user-related data comprises a first time corresponding to a first administration of the medical treatment for a predetermined time period, and a second time corresponding to a subsequent administration of the medical treatment for a predetermined time period, and wherein the mapping comprises determining a change in therapeutic effect between the first time and the second time.

9. The method of any preceding claim, wherein receiving user-related data comprising a time of administration of the medical treatment comprises receiving a dispensing time from an automatic treatment dispenser.

10. The method of any preceding claim, further comprising transmitting (518, 520) a result of determining the efficacy of the medical treatment to a server (408).

11. The method of any preceding claim, further comprising requesting and receiving the efficacy model corresponding to the medical treatment from a server, ready for mapping to the received sensor data.

12. The method of any preceding claim, further comprising controlling (514, 518, 520) a display (416) to output a result of determining the compliance to and efficacy of the medical treatment.

13. The method of any preceding claim, wherein receiving the sensor data from the personal care appliance comprises:
transmitting (602) a control signal to the personal care device to activate a secondary or auxiliary treatment function of the personal care appliance,
receiving (604) first sensor data corresponding to when the secondary or auxiliary treatment function is not active during usage of the personal care appliance, and
receiving (606) second sensor data corresponding to when the secondary or auxiliary treatment function is active during usage of the personal care appliance,
wherein mapping (608) the received sensor data to the efficacy model comprises mapping the first sensor data and second sensor data based on the received user-related data and usage time of the personal care appliance, and
determining (610) the efficacy of the medical treatment comprises comparing the mapping of the first sensor data to the mapping of the second sensor data.

14. An electronic apparatus (410) comprising at least one processor (412) configured to carry out the method of any of claims 1 to 13.

15. A transitory or non-transitory computer readable medium, having instructions stored thereon that, when executed by a processor, cause the processor to perform the method of any of claims 1 to 13.
